# EUROPEAN PATENT APPLICATION

(11) **EP 1 312 622 A1**
(43) Date of publication of application: **21.05.2003**
(21) Application number: 02079623.1
(22) Date of filing: 06.11.2002
(51) Int. Cl.: C08F 136/06, C08F 4/70, C08F 4/80

(54) **Process for the preparation of linear or branched 1,4-cis polybutadiene**

(30) Priority: 15.11.2001 IT MI20012409
(71) Applicant: ENI S.p.A., 00144 Roma (IT); Polimeri Europa S.p.A., 72100 Brindisi (IT)
(72) Inventor: Santi, Roberto, 28100 Novarra (IT); Sommazzi, Anna, 16038 Santa Margherita Ligure, Genova (IT); Ricci, Giovanni, 43100 Parma (IT); Masi, Francesco, 26866 Sant'Angelo Lodigano, Lodi (IT); Romano, Anna Maria, 28100 Novara (IT); Balducci, Alessandro, 48100 Ravenna (IT)
(74) Representative: De Gregori, Antonella

(57) **Abstract**

A process is described for the preparation of 1,4-cis polybutadiene by means of the polymerization of 1,3-butadiene, carried out in one or more solvents and in the presence of a catalytic system which comprises one or more catalysts selected from cobalt complexes, preformed or formed in situ, having general formula (I) Co^{(z)}L_{b}Wₘ
wherein (z), the cobalt oxidation number, is 2 or 3;
b = 1, 2, 3;
m = 0, 1, 2;
W is selected from Br, Cl, acetylacetonate, carboxylate, characterized in that:
L is a ligand having general formula (Ia)

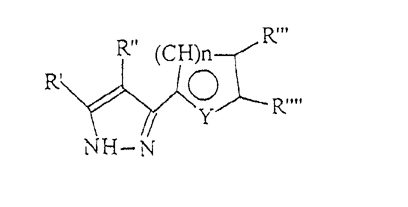

## Description

The present invention relates to a process for the preparation of 1,4-cis polybutadiene effected by means of the polymerization of 1,3-butadiene in the presence of a catalytic system comprising a catalyst consisting of cobalt complexed with particular ligands and a cocatalyst based on aluminum.

More specifically, the present invention relates to a process for the preparation of 1,4-cis polybutadiene carried out in the presence of a catalytic system which comprises (a) a catalyst consisting of cobalt complexed with particular ligands and (b) a cocatalyst selected from (b1) aluminoxanes and (b2) halogen aluminum alkyls selected from (III) AlRₙX₃₋ₙ (with n = 1 or 2) and (IV) Al₂RₙX₆₋ₙ (n = 1-5), wherein R is a C₁-C₂₀ alkyl group, X is chlorine or bromine, preferably chlorine. In the presence of aluminoxanes, a linear polybutadiene is obtained, which is particularly suitable for the preparation of tyres, whereas in the presence of halogen-derivatives (III) or (IV), a branched polybutadiene is obtained, which is particularly suitable for the preparation of HIPS high impact polystyrene.

Various types of catalysts and processes based on cobalt for the polymerization of 1,3-butadiene, have been developed. Some of these catalysts produce a polybutadiene which contains a high percentage of 1,4-cis units and which has excellent thermal and mechanical properties. Furthermore, the polybutadiene produced with catalysis based on cobalt, when the reaction is carried out in benzene in the presence of molecular weight regulators (for example butene-1), is gel-free and contains branchings which make it ideal for the modification of polystyrene.

Examples of catalytic systems based on cobalt carried out in benzene are described in various documents: US-A-3,135,725 describes catalysts based on cobalt salts and aluminum alkyls; US-A-3,046,265 describes catalysts based on aluminum alkyls, cobalt halides and acetyl halides; US-A-3,094,514- describes cobalt chloride-methylaluminum chloride; GB-A-916,383 describes cobalt octanoate-ethylaluminum chloride; GB-A-916,384 describes cobalt ethylhexanoateethylaluminum chloride; DE-A-1,495,935 describes cobalt octanoate-diethylaluminum chloride-water; GB-A-941,739 describes cobalt stearate-diethylaluminum chloride.

However, due to its carcinogen nature, benzene is not desired as a solvent following the restrictions imposed by OSHA (Occupational Safety and Health Agency) on the concentration of benzene allowed in work areas. The necessity is therefore felt for finding catalytic systems based on cobalt capable of operating with the same efficiency in aromatic solvents other than benzene and/or in solvents based on aliphatic hydrocarbons.

It has now been found that particular cobalt complexes overcome the above drawbacks.

In accordance with this, the present invention relates to a process for the preparation of 1,4-cis polybutadiene by means of the polymerization of 1,3-butadiene, carried out in one or more solvents and in the presence of a catalytic system which comprises one or more catalysts selected from cobalt complexes, preformed or formed in situ, having general formula (I)

Co^{(z)}L_{b}Wₘ (I)

wherein (z), the cobalt oxidation number, is 2 or 3;
b = 1, 2, 3;
m = 0, 1, 2;
W is selected from Br, Cl, acetylacetonate, carboxylate, with the constraint that:
when z = 2; m = 2, b = 1; W = Cl, Br, carboxylate;
when z = 3; m = 0, 1, 2; b = 1, 2, 3; W = acetylacetonate;
characterized in that:
L is a ligand having general formula (Ia) 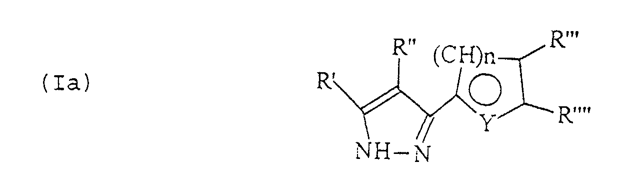 wherein
Y is selected from N, O and S
R' and R'', the same or different, are selected from hydrogen, linear or branched alkyl groups containing from 1 to 10 carbon atoms, cycloalkyl groups and aryl groups containing from 6 to 20 carbon atoms, wherein the above groups can be optionally halogenated, preferably with fluorine;
R''', R'''', the same or different, are selected from hydrogen, halogen, linear or branched C₁-C₂₀ alkyl groups, C₆-C₂₀ aryl groups, or they jointly form a condensed benzene ring, wherein said aryl groups or said condensed benzene ring can be optionally substituted with linear or branched alkyl groups containing from 1 to 10 carbon atoms,
n is an integer having the value of 1 or 2, preferably 2.

In the preferred embodiment, W is selected from Cl and acetylacetonate (acac).

Typical but non-limiting examples of ligands "L" are:
3-isopropyl-5-(2-pyridine)-pyrazole;
3-methyl-5-[2-(6-ter-butyl)-pyridine]-pyrazole;
3-trifluoromethyl-5-[2-(6-ter-butyl)-pyridine]-pyrazole;
3-trifluoromethyl-5-[2-(6-bromo)-pyridine]-pyrazole;
3-isopropyl-5-[2-(6-ter-butyl)-pyridine]-pyrazole;
3-perfluoropropyl-5-[2-(6-ter-butyl)-pyridine]-pyrazole;
3-isopropyl-5-[2-(6-(2,6-diisopropylphenyl))-pyridine]-pyrazole;
3-phenyl-5-[2-(6-(2,6-diisopropylphenyl))-pyridine]-pyrazole;
3-(2,6-diisopropyl-phenyl)-5-[2-(6-(2,6-diisopropylphenyl)-pyridine]-pyrazole;
3-isopropyl-4-methyl-5-[2-(4-ter-butyl-6-isopropyl)-pyridine]-pyrazole;
3-isopropyl-5-(2-furyl)-pyrazole;
3-isopropyl-5-[2-(5-ter-butyl)-furyl]-pyrazole;
3-isopropyl-5-[2-(5-(2,6-diisopropylphenyl))-furyl]-pyrazole;
3-isopropyl-5-[2-(7-ter-butyl)-benzofuryl]-pyrazole;
3-isopropyl-4-methyl-5-[2-(7-ter-butyl)-benzofuryl]-pyrazole;
3-trifluoromethyl-5-[2-(5-(2,6-diisopropyl-phenyl))-furyl]-pyrazole;
3-isopropyl-4-methyl-5-[2-(5-(2,6-diisopropyl-phenyl))-furyl]-pyrazole;
3-perfluorobutyl-4-methyl-5-[2-(5-(2,6-diisopropyl-phenyl))-furyl]-pyrazole;
3-phenyl-4-methyl-5-[2-(5-(2,6-diisopropyl-phenyl))-furyl]-pyrazole;
3-isopropyl-5-(2-thiophene)-pyrazole;
3-isopropyl-5-[2-(5-terbutyl)-thiophene]-pyrazole;
3-isopropyl-5-[2-(5-(2,6-diisopropylphenyl))-thiophene]-pyrazole;
3-isopropyl-5-[2-(7-ter-butyl)-benzothiophene]-pyrazole;
3-isopropyl-4-methyl-5-[2-(7-ter-butyl)-benzothiophene]-pyrazole;
3-trifluoromethyl-5-[2-(5-(2,6-diisopropyl-phenyl))-thiophene-pyrazole;
3-isopropyl-4-methyl-5-[2-(5-(2,6-diisopropyl-phenyl))-thiophene]-pyrazole;
3-perfluorobutyl-4-methyl-5-[2-(5-(2,6-diisopropyl-phenyl))-thiophene]-pyrazole;
3-phenyl-4-methyl-5-[2-(5-(2,6-diisopropyl-phenyl))-thiophene]-pyrazole;

The structures claimed of typical ligands, subdivided for the sake of convenience, into three groups, are provided below for illustrative purposes but, however, are non-limiting.

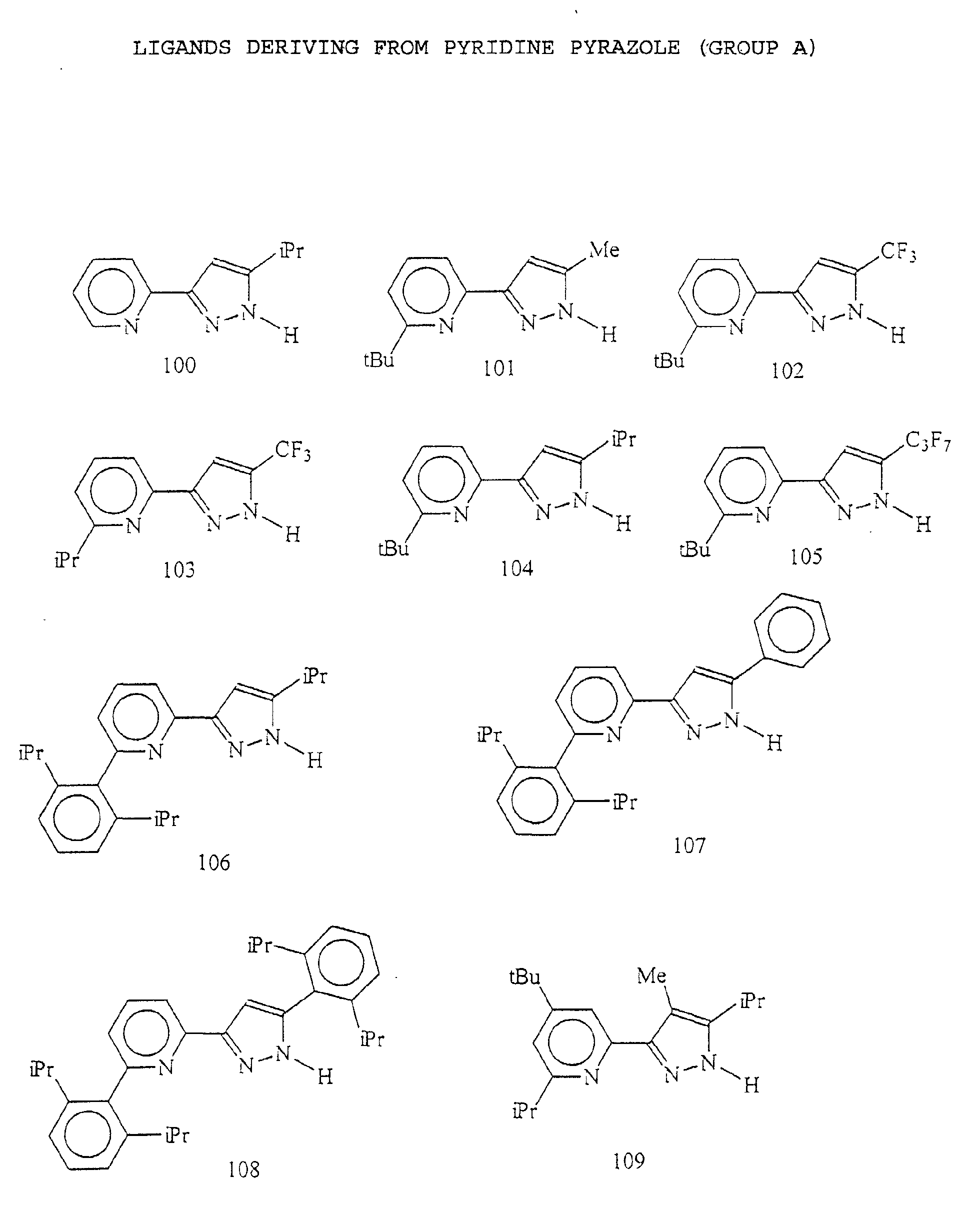

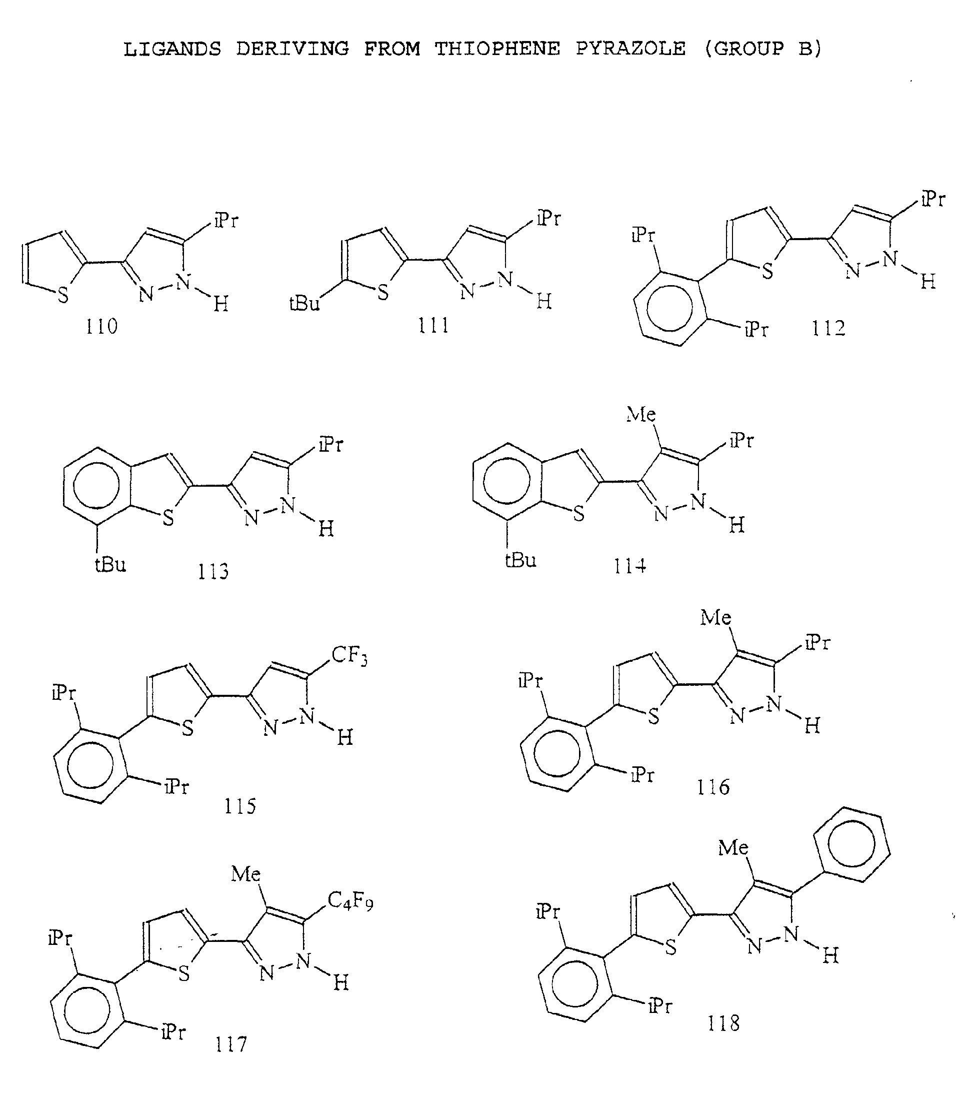

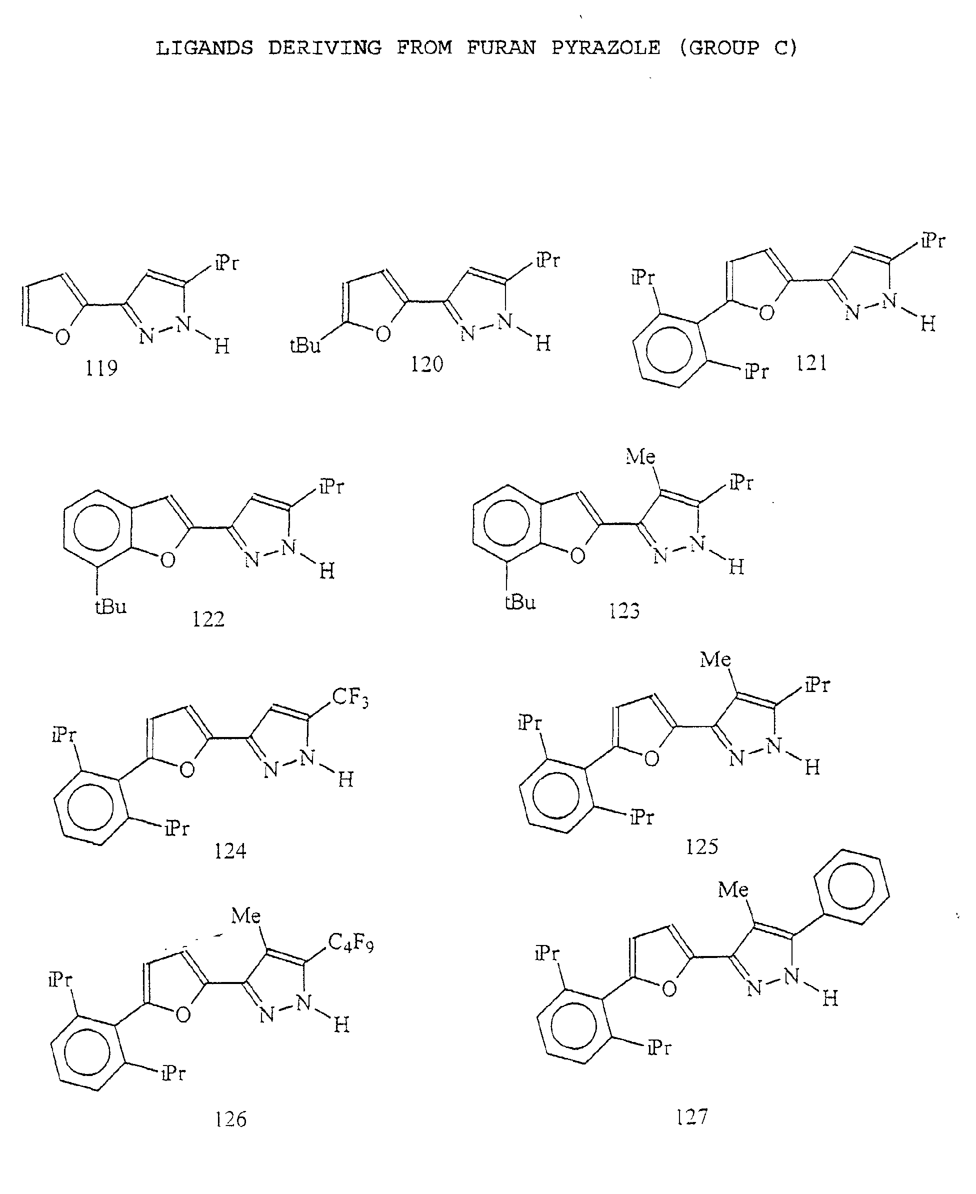

Typical but non-limiting examples of cobalt complexes having general formula (I) are:
Cobalt-[3-trifluoromethyl-5-[2-(6-bromo)-pyridine]-pyrazolyl] -dichloride;
Cobalt-[3-methyl-5-[2-(6-ter-butyl)-pyridine]-pyrazolyl]-dichloride;
Cobalt-[3-trifluoromethyl-5-[2-(6-ter-butyl)-pyridine]-pyrazolyl]-dichloride;
Cobalt-[3-phenyl-5-[2-(6-(2,6-diisopropylphenyl)-pyridine]-pyrazolyl]-dichloride;
Cobalt-[3-isopropyl-5-(2-furyl)-pyrazolyl]-dichloride;
Cobalt-[3-isopropyl-5-[2-(7-ter-butyl)-benzofuryl)-pyrazolyl]-dichloride;
Cobalt-[3-isopropyl-4-methyl-5-[2-(7-ter-butyl)-benzofuryl)-pyrazolyl]-dichloride;
Cobalt-[3-trifluoromethyl-5-[2-(5-(2,6-diisopropyl-phenyl))-furyl]-pyrazolyl]-dichloride;
Cobalt-[3-isopropyl-5-(2-thiophene)-pyrazolyl]-dichloride;
Cobalt-[3-isopropyl-5-[2-(7-ter-butyl)-benzothiophene)-pyrazolyl]-dichloride;
Cobalt-[3-isopropyl-4-methyl-5-[2-(7-ter-butyl)-benzothiophene)-pyrazolyl] -dichloride;
Cobalt-[3-trifluoromethyl-5-[2-(5-(2,6-diisopropyl-phenyl))-thiophene-pyrazolyl]-dichloride;
Cobalt-[3-trifluoromethyl-5-[2-(6-bromo)-pyridine]-pyrazolyl]-bis-pentan-2,4-dienoate;
Cobalt-[3-methyl-5-[2-(6-ter-butyl)-pyridine]-pyrazolyl]-bis-pentan-2,4-dienoate;
Cobalt-[3-phenyl-5-[2-(6-(2,6-diisopropylphenyl)-pyridine]-pyrazolyl]-bis-pentan-2,4-dienoate;
Cobalt-tris-[3-(2-pyridine)-5-trifluoromethyl-pyrazole];
Cobalt-bis-[3-isopropyl-5-(2-furyl)-pyrazolyl]-pentan-2,4-dienoate;
Cobalt-[3-isopropyl-5-[2-(7-ter-butyl)-benzofuryl)-pyrazolyl]-bis-pentan-2,4-dienoate;
Cobalt-[3-isopropyl-4-methyl-5-[2-(7-ter-butyl)-benzofuryl]-pyrazolyl]-bis-pentan-2,4-dienoate;
Cobalt-[3-trifluoromethyl-5-[2-(5-(2,6-diisopropyl-phenyl))-furyl)]-pyrazolyl]-bis-pentan-2,4-dienoate;
Cobalt-bis-[3-isopropyl-5-(2-thiophene)-pyrazolyl]-pentan-2,4-dienoate;
Cobalt-[3-isopropyl-5-[2-(7-ter-butyl)-benzothiophene]-pyrazolyl]-bis-pentan-2,4-dienoate;
Cobalt-[3-isopropyl-4-methyl-5- [2- (7-ter-butyl) -benzothiophene]-pyrazolyl]-bis-pentan-2,4-dienoate;
Cobalt-[3-trifluoromethyl-5-[2-(5-(2,6-diisopropyl-phenyl))-thiophene)]-pyrazolyl]-bis-pentan-2,4-dienoate.

Typical but non-limiting structures of cobalt complexes having general formula (I) are:

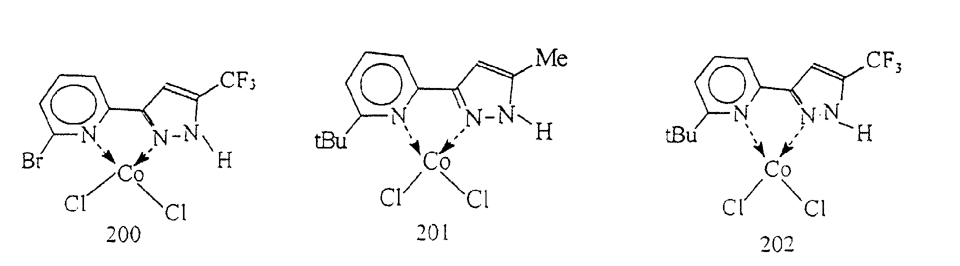

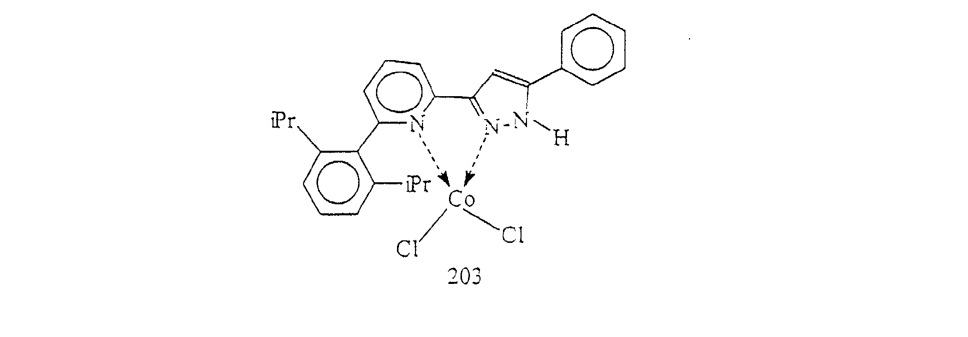

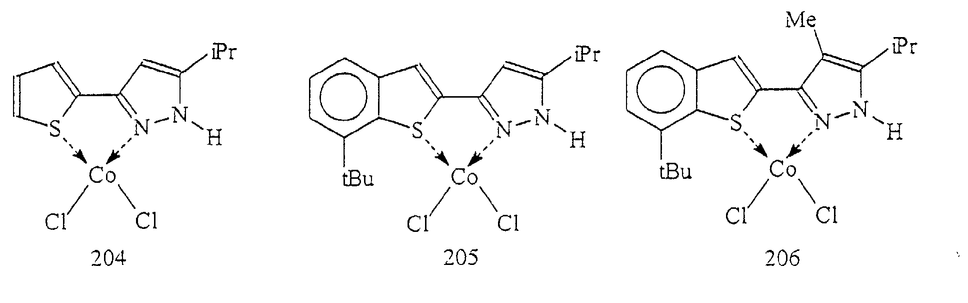

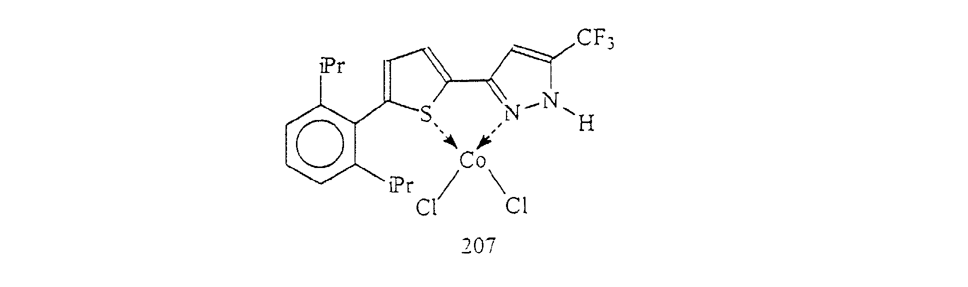

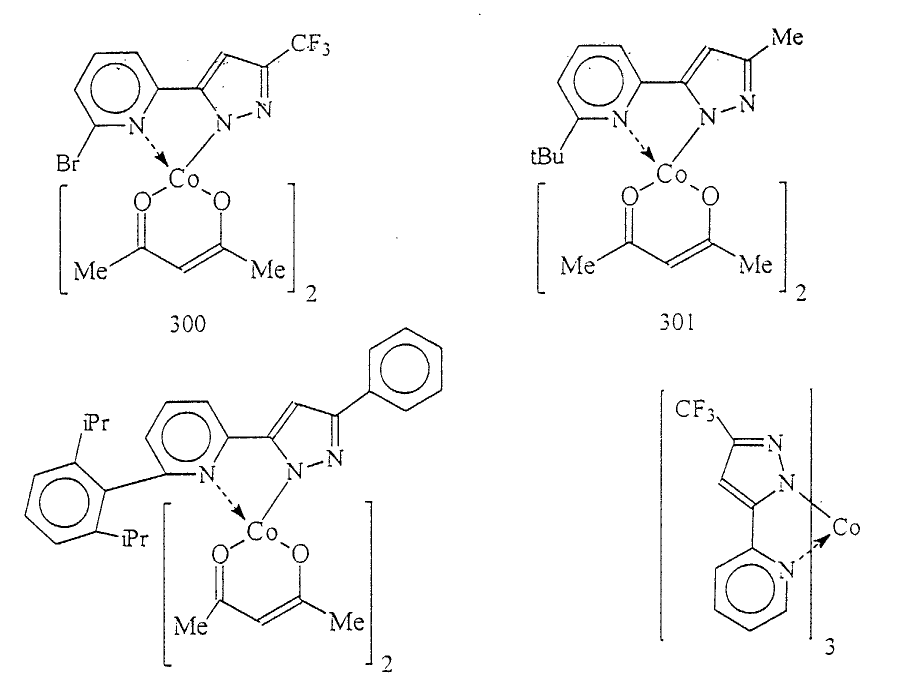

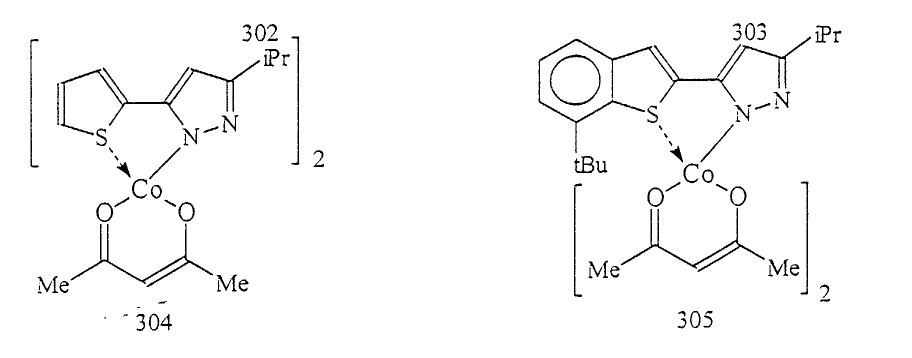

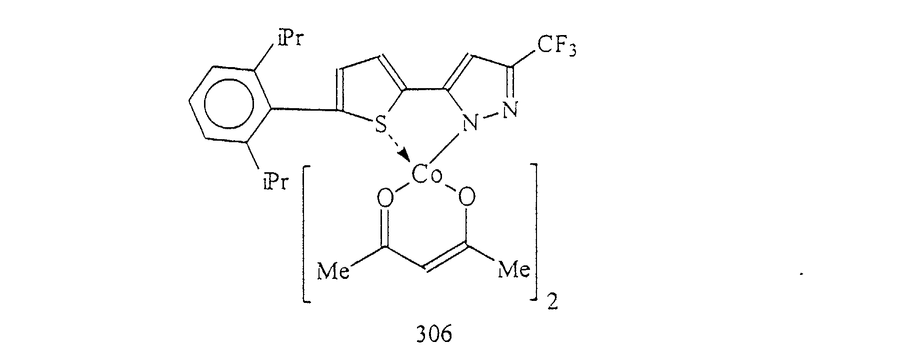

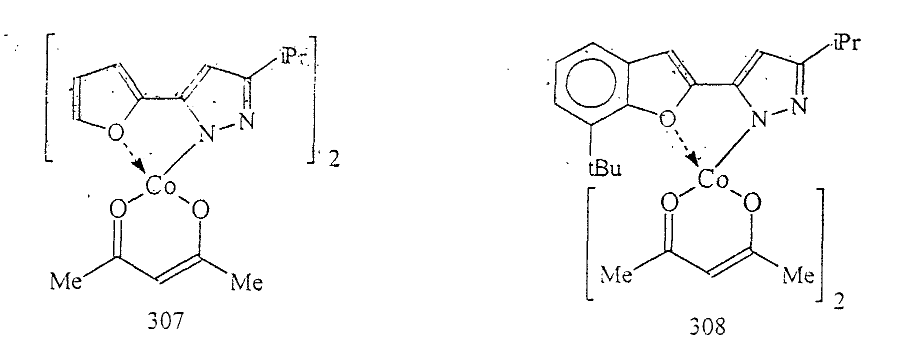

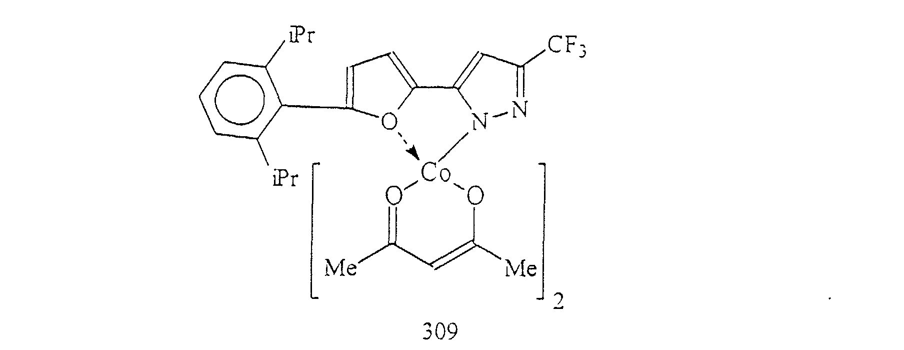

The term "1,4-cis polybutadiene " refers to polybutadiene in which at least 90% of the butadiene units is concatenated in 1,4-cis position.

The term "branched polybutadiene" refers to a polybutadiene having a g value (defined further on in the experimental part) ≤ 0.90, preferably ≤ 0.85. This branched polybutadiene is particularly suitable for the preparation of HIPS (high impact polystyrene).

The term "linear polybutadiene" refers to polybutadiene having a g value ≥ 0.95. This linear polybutadiene is particularly suitable for the preparation of elastomeric blends for tyres.

The complexes having general formula (I) can be synthesized according to known procedures, for example by reaction of the cobalt salt selected (for example CoCl₂ or Co(acac)₃) with the desired ligand in the stoichiometric ratio, in a hydrocarbon solvent or in alcohol.

The catalytic system of the present invention also comprises, in addition to the cobalt complex having general formula (I), one or more cocatalysts selected from organo-derivatives of aluminum selected from:
(b1) aluminoxanes and relative derivatives.
(b2) halogen aluminum alkyls selected from (III) AlRₙX₃₋ₙ (with n = 1 or 2) and (IV) Al₂RₙX₆₋ₙ (n = 1-5), wherein R is a C₁-C₂₀, preferably C₁-C₅, alkyl group, X is chlorine or bromine, preferably chlorine.

When a linear 1,4-cis polybutadiene is desired, the organo-derivative of aluminum is selected from aluminoxanes and the relative derivatives (b1).

When a branched 1,4-cis polybutadiene is desired, the organo-derivative of aluminum is selected from halogen aluminum alkyls (b2), particularly from those having general formula (IV) Al₂RₙX₆₋ₙ.

In accordance with this, the present invention also relates to a process for obtaining linear 1,4-cis polybutadiene by means of the polymerization of 1,3-butadiene in the presence of one or more solvents and a catalytic system, characterized in that said catalytic system comprises:
(a) a catalyst selected from one or more cobalt complexes, preformed or formed in situ, having general formula (I) CO^{(z)}L_{b}Wₘ, wherein z, b, m, L, W have the meaning defined above,
(b) a cocatalyst selected from aluminoxanes and relative derivatives.

The present invention also relates to a process for obtaining branched 1,4-cis polybutadiene by means of the polymerization of 1,3-butadiene in the presence of one or more solvents and a catalytic system, characterized in that said catalytic system comprises:
(a) a catalyst selected from one or more cobalt complexes, preformed or formed in situ, having general formula (I) Co^{(z)}L_{b}Wₘ, wherein z, b, m, L, W have the meaning defined above,
(b) a cocatalyst selected from halogen aluminum alkyls having general formula (III) AlRₙX₃₋ₙ (with n = 1 or 2) or (IV) Al₂RₙX₆₋ₙ (n = 1-5), wherein R is a C₁-C₂₀ alkyl group, X is chlorine or bromine, preferably chlorine.

In the preferred embodiment, the cocatalyst (b) is selected from compounds having general formula (IV) Al₂RₙX₆₋ₙ (n = 1-5). In an even more preferred embodiment, the cocatalyst is Al₂(C₂H₅)₃Cl₃.

As far as the aluminoxanes (b1) are concerned, it is known that these are compounds containing Al-O-Al bonds, with a varying O/Al ratio, obtained by the reaction, under contolled conditions, of an aluminum alkyl, or aluminum alkyl halide, with water or other compounds containing preestablished quantities of available water, as, for example, in the case of the reaction of aluminum trimethyl with aluminum hexahydrate sulfate, copper pentahydrate sulfate or iron pentahydrate sulfate. Aluminoxanes preferably used for the formation of the polymerization catalyst of the present invention are cyclic and/or linear, oligo- or polymeric compounds, characterized by the presence of repetitive units having the following formula: 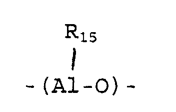 wherein R₁₅ is a C₁-C₆ alkyl group, preferably methyl. Each aluminoxane molecule preferably contains from 4 to 70 repetitive units which may also not be all the same, but contain different R₁₅ groups.

With respect to the halogen aluminum alkyls (b2), these have general formula (III) AlRₙX₃₋ₙ (with n = 1 or 2) or (IV) Al₂RₙX₆₋ₙ (n = 1-5), wherein R is a C₁-C₂₀ alkyl group, X is chlorine or bromine, preferably chlorine. Typical examples of compounds having general formula (III) are AlEt₂Cl (diethyl aluminummonochloride), AlMe₂Cl (dimethylaluminummonochloride), AlEtCl₂ (ethylaluminumdichloride), Al(i-bu)₂Cl (diisobutylaluminummonochloride); typical examples of compounds having general formula (IV) are Al₂Et₃Cl₃ (ethylaluminumsesquichloride), Al₂Me₃Cl₃ (methylaluminumsesquichloride).

In the process of the present invention, for the production of both linear and branched polybutadiene, the molar ratio between the complex having general formula (I) and the aluminum of the cocatalyst ranges from 1:5 to 1:10000, preferably from 1:10 to 1:1000.

The cobalt complex having general formula (I) can be used in a molar ratio with respect to the 1,3-butadiene ranging from 1/10⁵ to 1/10², preferably from 1/2x10⁴ to 1/10³.

When the preformed complex having general formula (I) is not used, but rather the formation technique in situ, it is possible to use a molar ratio between ligand (L) and cobalt salt from at least 1/1 to 3/1. It is obviously possible to operate with higher ratios.

As far as the polymerization of butadiene is concerned, the above polymerization is preferably effected in a polymerization medium comprising an inert hydrocarbon which is a solvent of butadiene and of the catalytic system. Inert hydrocarbons which can be used in the polymerization process comprise aliphatic hydrocarbons, olefins included, cycloaliphatic, aromatic hydrocarbons and relative mixtures. More specifically, suitable hydrocarbons are those selected within the group of C₄ to C₈ aliphatic hydrocarbons, olefins included, within the group of C₅ to C₁₀ cycloaliphatic hydrocarbons, and relative mixtures. Typical, non-limiting examples of the above hydrocarbons are butane, pentane, hexane, heptane, cyclopentane, cyclohexane, butenes. The use of the above aliphatic and cycloaliphatic solvents is particularly advisable as it reduces problems relating to environmental impact. The process of the present invention can also be carried out in the presence of aromatic solvents, particularly toluene, as such or mixed with aliphatic solvents.

The concentration of 1,3-butadiene in the polymerization medium can vary in relation to the particular solvent medium or diluent used. When solvents are used in which both 1,3-butadiene and also the polymeric product are soluble, the concentration of 1,3-butadiene preferably ranges from 10 to 35% by weight with respect to the total weight of the mixture.

The polymerization temperature of 1,3-butadiene preferably ranges from -30°C to +60°C, the lower temperature limit being determined more by the freezing point of the reaction mixture rather than by the catalytic activity. More preferably, the polymerization process is carried out at a temperature ranging from -10°C to +40°C.

The process of the present invention can also be carried out (see the experimental part) in the absence of the usual molecular weight regulators well-known to experts in the field, for example butene-1. This represents another significant advantage of the process of the present invention. In the process of the present invention, in fact, the molecular weight control can be effected by varying the type of cobalt complex and/or ratio between the aluminum compound and cobalt.

The polymerization reaction can be stopped by the addition of one or more polymerization terminators which deactivate the catalytic system, followed by the conventional solvent-removal (desolventizing), washing and drying phases, which are normal operations in the production of polydienes. The terminator used for deactivating the catalytic system is typically a protic compound, which includes, but is not limited to, an alcohol, a carboxylic acid, an inorganic acid, and water or a combination thereof. An antioxidant such as 2,6-di-ter-butyl-4-methylphenol can be added, before, after or with the addition of the terminator. The quantity of antioxidant usually ranges from 0.2% to 1% by weight with respect to the polymer.

At the end of the polymerization, the polybutadiene can be recovered according to the standard techniques, preferably by means of the coagulation technique. Any possible residues of the solvent can be removed from the polymer by means of evaporation, which can be facilitated by high temperatures and vacuum application.

In addition to the above advantages, the process of the present invention allows polybutadiene to be produced with a reduced gel content, particularly macro gels. This is particularly important in the case of branched 1,4-cis polybutadiene as it allows the use of the latter for the preparation of HIPS impact polystyrene.

The following examples are provided for a better understanding of the present invention.

### EXAMPLE 1: Synthesis of 1-(2-pyridine)-4,4,4-trifluoromethylbutan-1,3-dione

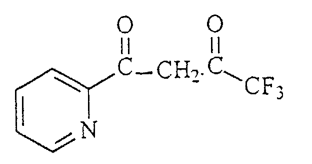

- CF₃COOC₂H₅ 42.6 g = 0.3 mol
- NaH 12 g = 0.3 mol
- 2-acetylpyridine 18.3 g = 0.15 mol
- anhydrous Et₂O 300 ml

250 ml of ethyl ether are added, under nitrogen, in a three-necked flask, to 36.5 ml of CF₃COOC₂H₅, and NaH. 17 ml of 2-acetylpyridine are added dropwise to 50 ml of ethyl ether, the suspension being maintained at reflux temperature, under stirring for 6 hours. 100 ml of ethyl ether are subsequently added, the mixture is left for a further hour at reflux temperature and for the whole night under stirring at room temperature. 50 ml of ethanol are added to the pink-coloured suspension, which is left under stirring for 1 hour. The suspension is poured into a mixture of water and ice (150 ml of H₂O/150 g of ice). The two phases are separated and the aqueous phase is washed with Et₂O (2 x 100 ml). The aqueous phase is acidified with H₂SO₄ at 10%, up to neutral pH, and is then extracted with Et₂O. The ether phase is anhydrified with Na₂SO₄, the solvent is evaporated and dried with a mechanical pump. A reddish-brown oil is obtained (27.7 g = 0.13 moles). Yield 86.6%.
M.W. = 217.15 g/mol
¹H NMR (CDCl₃) : 8.71 (1H, m), 8.17 (1H, m) , 7.95 (1H, m) , 7.63 (1H, m), 3.55 (2H)

### EXAMPLE 1b: Synthesis of 3-trifluoromethyl-5-pyridinepyrazole

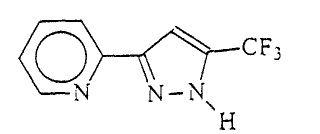

- diketone of example (1a) 27.7 g = 0.13 mol
- NH₂NH₂·H₂O 25 g = 0.5 mol
- EtOH 500 ml
- H₂O 20 ml

β-diketone is dissolved in ethanol, under nitrogen, in a three-necked flask. 24 ml of hydrazine hydrate in 20 ml of water are added, dropwise in about one hour. The mixture is left at reflux temperature for an hour. It is left to cool and is diluted in 500 ml of H₂O. It is then extracted with Et₂O (6 x 200 ml). The ether phase is washed with H₂O (2 x 100 ml)and anhydrified on Na₂SO₄. The solvent is evaporated and the pink solid is dried with a mechanical pump obtaining 26 g of raw product, which is crystallized from 200 ml of hot toluene. 12.3 g of pink solid are obtained, which is dried with a mechanical pump.
M.W. = 213.16 g/mol
¹H NMR (C₂D₆O) : 8.50 (1H, d), 7.95 (1H, d), 7.81 (1H, m) , 7.33 (1H, m), 3.41 (2H, s).
IR spectrum (nujol) : ν_{N-H} = 3275.81 cm⁻¹, ν_{C-F} = 1168.96 cm⁻¹

### EXAMPLE 2a: Synthesis of 1-(2-thiophene)-4,4,4-trifluoromethylbutan-1,3-dione

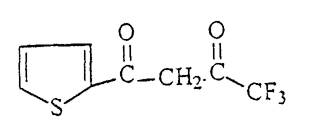

- CF₃COOC₂H₅ 42.6 g = 0.3 mol
- NaH 12 g = 0.3 mol
- 2-acetylthiophene 19.3 g = 0.15 mol
- anhydrous Et₂O 300 ml

250 ml of ethyl ether are added, under nitrogen, in a three-necked flask, to 36.5 ml of CF₃COOC₂H₅, and NaH. 16.5 ml of 2-acetylthiophene are added dropwise to 50 ml of ethyl ether, the suspension being maintained at reflux temperature, under stirring for 5 hours. 100 ml of ethyl ether are subsequently added; the mixture is left for a further hour at reflux temperature and for the whole night under stirring at room temperature. 50 ml of ethanol are added to the pink-coloured suspension, obtaining a yellow solution, which is left under stirring for 1 hour. The solution is poured into a mixture of ice and water acidified with H₂SO₄ (150 ml of H₂O/150 g of ice). The two phases are separated and the aqueous phase is washed with Et₂O (2 x 100 ml). The aqueous phase is acidified with H₂SO₄ at 10%, up to neutral pH, and is then extracted with Et₂O. 17.25 g of pink solid are obtained.
M.W. = 222.18 g/mol
¹H NMR (ether phase, CDCl₃): 7.81 (1H, d), 7.73 (1H, d), 7.16 (1H, m), 6.14 (1H, s).

### EXAMPLE 2b: Synthesis of 3-trifluoromethyl-5-thiophenepyrazole

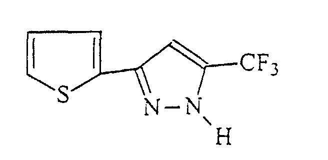

- diketone of example (2a) 17.3 g = 0.078 mol
- NH₂NH₂·H₂O 11.7 g = 0.23 mol
- EtOH 200 ml
- H₂O 20 ml

β-diketone is dissolved in ethanol, under nitrogen, in a three-necked flask. Hydrazine hydrate in 20 ml of water are added, dropwise in about one hour. The mixture is left at reflux temperature for an hour. It is left to cool and is diluted in 200 ml of H₂O. It is then extracted with Et₂O (6 x 200 ml). The ether phase is washed with H₂O (2 x 100 ml) and anhydrified on Na₂SO₄. The solvent is evaporated and the solid is dried with a mechanical pump. 16.3 g of yellow solid are obtained.
M.W. = 218.20 g/mol
¹H NMR (CDCl₃): 7.52 (1H, m), 7.36 (1H, m), 7.10 (1H, m), 6.68 (1H, s).

### EXAMPLE 3a: Synthesis of 1-(2-benzofuranyl)-4,4,4-trifluoromethyl-butane-1,3-dione.

35.7 ml of 2-acetyl benzofuran (0.30 moles) are added dropwise, in about 4 hours, to a suspension of 35.7 ml of ethyl trifluoroacetate (0.60 moles) and 24.0 g of NaH 60% (0.60 moles) in 500 ml of anhydrous ethyl ether. A further 500 ml of anhydrous ethyl ether are added to keep the reaction mass fluid. The mixture is refluxed for an hour, and after this period, 50 ml of ethanol are slowly added. The reaction mixture is poured into a 1 litre beaker containing 500 grams of ice and water, and HCl is added up to pH = 5. The two phases are then separated and the aqueous phase is washed with ethyl ether (3 x 200 ml), whereas the ether phase, after washing with NaHCO₃ until neutrality, is washed again with water, dried on Na₂SO₄ and then evaporated from the solvent. 26.4 g of product are obtained, which upon NMR analysis proves to correspond to a mixture of keto-enolic tautomers. (Yield:42%).
¹H NMR (in CD₃COCD₃, ppm) : 7.66 (2H, m) , 7.55 (2H, m) , 6.20 (1H, s), 3.20 (2H, s).

### EXAMPLE 3b: Synthesis of 3-trifluoromethyl-5-benzofuranyl pyrazole

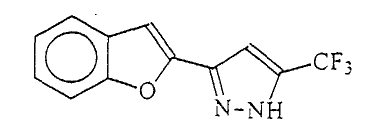

20 g of hydrazine hydrate (0.4 moles) in water, are slowly added dropwise to a solution containing 26 ml of 1-(2-benzofuranyl)-4,4,4-trifluoromethylbutan-1,3-dione (0.128 moles) in 500 ml of ethyl ether, with a slight development of heat. At the end of the addition, the mixture is refluxed for an hour. The two phases are then separated and the aqueous phase is washed with ethyl ether (6 x 200 ml), whereas the organic phase, after being washed with NaHCO₃ to neutrality and again with water, is dried on Na₂SO₄ and then evaporated from the solvent. 16.0 g of solid are obtained.
¹H NMR (in CD₃COCD₃, ppm) : 7.46 (2H, q) , 7.25 (2H, q), 6.92 (1H, s), 6.32 (1H, s). mass: 252 (molecular ion).

### EXAMPLE 4: Synthesis of [3-trifluoromethyl-5-pyridinepyrazole]cobalt dichloride

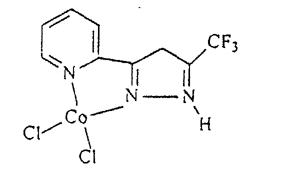

- CoCl₂·6H₂O 0.33 g = 1.4 mmol
- 3-trifluoromethyl-5-pyridinepyrazole 0.29 g = 1.4 mmol
- n-butanol 8 ml

The CoCl₂·6H₂O is dissolved in n-butanol and the ligand dissolved in n-butanol is added, obtaining a greyish-blue solution. The mixture is left under stirring at T = 80°C for 1/2 hour. It is left to cool to room temperature, but no change is observed. Upon the addition of hexane, a lilac-coloured precipitate is formed. The precipitate is filtered under vacuum on a gooch 4 and is washed with Et₂O. 0.37 g of product are obtained. Yield 77%.
M.W. = 343.09 g/mol

| Elemental analysis (% by weight) | | |
|---|---|---|
| Theoretical | Co = 17.2 | Cl = 20.6 |
| Real | Co = 15.8 | Cl = 18.7 |

IR spectrum (nujol) : ν_{N-H} = 3308.95 cm⁻¹, ν_{C=N} = 1609.5 cm⁻¹, ν_{C-F} = 1129.31 cm⁻¹

### EXAMPLE 5: Synthesis of cobalt [3-(2-pyridine)-5-trifluoromethyl-pyrazole]-bis-acetylacetonate

0.19 g (0.9 mmoles) of 3-(2-pyridine)-5-trifluoromethyl-pyrazole dissolved in 10 ml of toluene are added to a solution of 0.31 g (0.9 mmoles) of Co(AcAc)₃ in 10 ml of toluene. The mixture is left under stirring at 25°C for 1 hour and the solution is then concentrated to 5 ml, cooled to -10°C for 48 hours; the green solid precipitated is filtered and subsequently dried at 25°C and 10⁻³ mmHg. 0.20 g (47% yield) of complex are obtained.
Elemental analysis
Theoretical (%) for C₁₉H₁₉CoF₃N₃O₄: Co = 12.56, C = 44.63, H = 4.08, N = 8.95.
Experimental (%): Co = 12.9; C = 45.0; H = 3.85; N = 9.01

### EXAMPLE 6: Synthesis of cobalt-bis-[3-(2-pyridine)-5-trifluoromethyl-pyrazole]-acetylacetonate

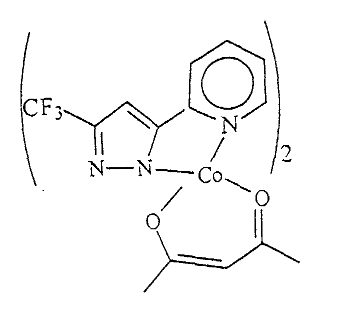

0.384 g (1.8 mmoles) of 3-(2-pyridine)-5-trifluoromethyl-pyrazole dissolved in 10 ml of toluene are added to a solution of 0.31 g (0.9 mmoles) of Co(AcAc)₃ in 10 ml of toluene. The temperature is brought to 70°C and the mixture is maintained at this value for 1 hour; it is then cooled to room temperature and the solution is concentrated to 10 ml. It is subsequently cooled to -10°C and maintained at this temperature for 48 hours; the green solid obtained is filtered and subsequently dried at 25°C and 10⁻³ mmHg. 0.25 g (24% yield) of complex are obtained.
Elemental analysis
Theoretical (%) for C₂₃H₁₇CoP₆N₆O₂: Co = 10.12, C = 47.44, H = 2.94, N = 14.43
Experimental (%): Co = 10.9; C = 47.0; H = 3.20; N = 15.0

### EXAMPLE 7: Synthesis of cobalt-tris-[3-(2-pyridine)-5-trifluoromethyl-pyrazole]

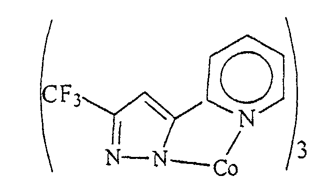

0.57 g (2.7 mmoles) of 3-(2-pyridine)-5-trifluoromethyl-pyrazole dissolved in 15 ml of toluene are added to a solution of 0.31 g (0.9 mmoles) of Co(AcAc)₃ in 10 ml of toluene. The temperature is brought to 70°C and the mixture is maintained at this value for 2 hours; it is then cooled to room temperature and the green solid precipitated is filtered and subsequently dried at 25°C and 10⁻³ mmHg. 0.40 g (63% yield) of complex are obtained.
Elemental analysis
Theoretical (%) for C₂₇H₁₅CoF₉N₉: Co = 8.47, C = 46.63, H = 2.17, N = 18.13
Experimental (%): Co = 8.9; C = 46.20; H = 2.35; N = 18.7

### POLYMERIZATION TESTS

Butadiene (10 ml) and subsequently toluene (90 ml) are condensed in a 200 ml reactor, maintained at a temperature of -20°C. The reactor is brought to the desired polymerization temperature (about 20°C); the aluminum compound is then charged (1 x 10⁻² moles of MAO or Al₂Et₃Cl₃ depending on the case) and finally a toluene solution of cobalt (preformed cobalt complex or toluene solution containing the non-complexed cobalt compound + ligand in the desired ratio), the quantity of cobalt being 1 x 10⁻⁵ moles. The whole mixture is kept under stirring for about 60 minutes.

The polymerization is terminated by adding methanol containing minimum quantities of hydrochloric acid; the polymer is coagulated with methanol containing small quantities of antioxidant, washed several times with methanol and then dried under vacuum at room temperature.

### Analytical procedures

### NMR ANALYSIS

The ¹H and ¹³C NMR spectra were registered with a Bruker AM 270 MHz spectrophotometer. The spectra were obtained in CDCl₃ at room temperature using tetramethylsilane as internal standard or in high temperature C₂D₂Cl₄ (103°C; HMDS as internal standard). The concentration of the polymeric solutions is about 10% by weight.

The microstructure of the polybutadienes obtained was determined on the basis of what is already known in literature (see for example V.D. Mochel, J. Polym. Sci, A-1 10, 1009, 1972; K.F. Elgert, G. Quack, B. Stutzel, Makromol. Chem. 175, 1955, 1974; D. Morero, A. Santambrogio, L. Porri, F. Ciampelli, Chim. Ind. (Milan) 41, 758, 1958).

### G.P.C. ANALYSIS

Operating conditions:
- Agilent 1100 pump
- Agilent 1100 I.R. Detector
- PL Mixed-A Columns
- Solvent/Eluant THF
- Flow 1 ml/min
- Temperature 25°C
- Molecular mass calculation: Universal Calibration method

### G.P.C./MALLS/I.R. ANALYSES

Operating conditions:
- Agilent 1050 pump
- Agilent 1050 I.R. Detector
- MALLS Dawn-DSP Wyatt - Technology Detector, λ=632.8 nm
- PL 10⁵-10⁵-10⁴-10³ Columns
- Solvent/Eluant THF
- Flow 1 ml/min
- Temperature 25°C
- Average g branching index calculation: ratio between radius of gyration of the branched macromolecule and that of the linear macromolecule, with the same molecular weight; the ratio is effected for each point of the integrated chromatogram, after which the average on all the points is effected. A polybutadiene is defined as branched when g ≤ 0.90.

### VISCOSITY MEASUREMENTS IN STYRENE

- Solvent : Styrene
- Concentration : 5% w/w
- Capillary: Cannon -Fenske series 200
- Calculation of the viscosity η = kt
wherein k = capillary constant; d = styrene density; t = fall time inside the capillary.
Viscosity values lower than or equal to 100 cP are acceptable for the modification of polystyrene.

### DETERMINATION OF THE GEL CONTENT

- Solvent : THF
- Concentration : 0.3% w/w
- Insoluble macro: 325 mesh net;
- Insoluble micro: 0.2 µm Teflon filter.
The insoluble % is gravimetrically determined, by weighing the residue on each filter and expressing it in percentage with respect to the initial weight.

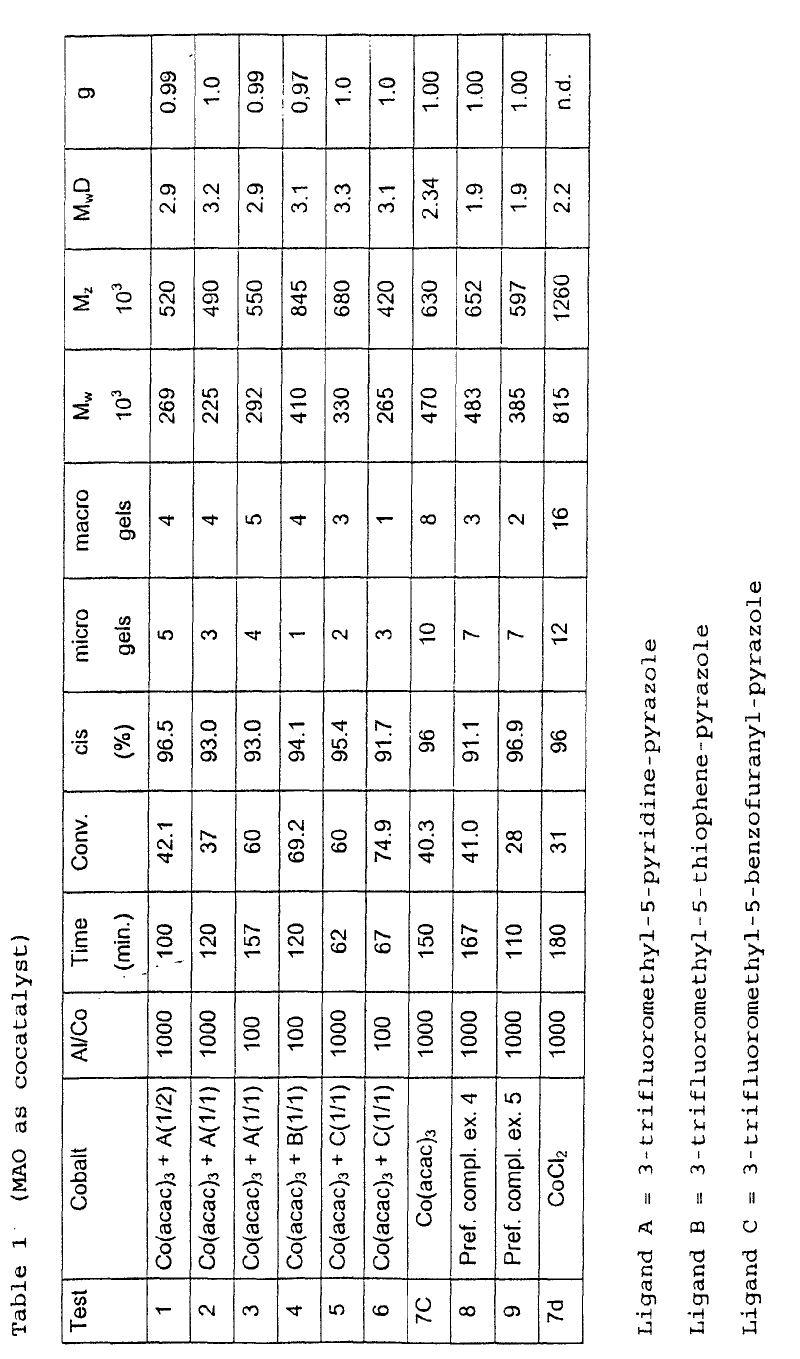

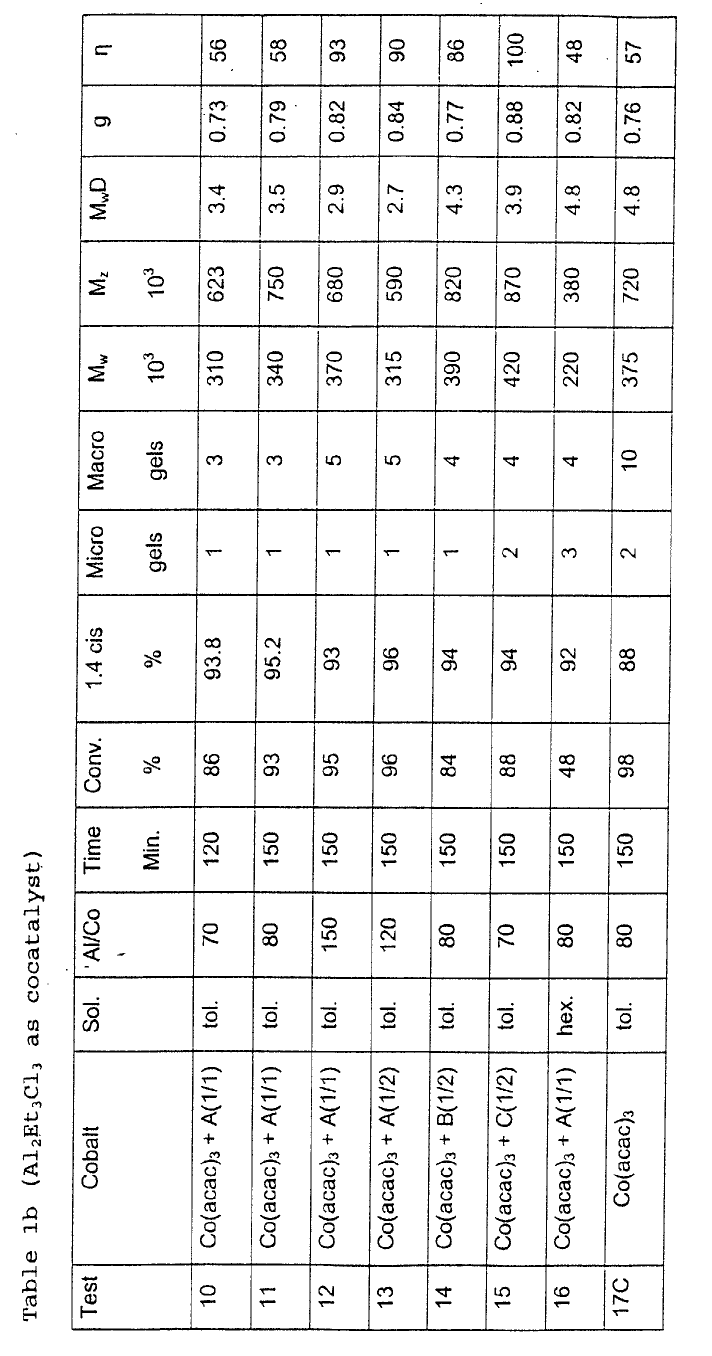

### Comments on the tables

From table 1a (tests with MAO), it can be seen how the process of the present invention, carried out in the presence of cobalt complexes formed in situ (tests 1-6), or preformed (tests 8 and 9), produce linear 1,4-cis polybutadiene. The polybutadiene, in fact, has a content of 1,4 cis units higher than 91%, in most cases from 93% to 96%, whereas the g value (which indicates linearity) ranges from 0.97 to 1.00. Furthermore, all the tests effected according to the process of the present invention show a low gel content (both micro and macro). Comparative examples 7c and 7d, on the contrary, carried out in the absence of the ligands of the present invention, show a polybutadiene which is still linear (g = 1.00) but with an unacceptable gel content.

All the tests from 1 to 6 provide linear 1,4-cis polybutadiene with molecular weights which are commercially acceptable for application in the tyre industry.

With respect to the tests effected in the presence of Al₂Et₃Cl₃ (table 1b), it can be observed that this cocatalyst always produces 1,4-cis polybutadienes, but branched. This is demonstrated by the "g" values which are all lower than 0.90.

The gel content, furthermore, (micro ≤ 3, macro ≤ 5) is acceptable and the viscosity in styrene is equal to or lower than 100 cP, therefore allowing the polybutadiene thus obtained, to be used in the preparation of impact polystyrene (HIPS). It should be pointed out that comparative example 17C (absence of the ligand) has a low content of 1,4-cis units and a high content of macro gels. Table 1b also indicates the possibility of carrying out the process with the use of an aliphatic solvent (hexane).

In conclusion, the catalytic system of the present invention allows linear or branched 1,4-cis polybutadiene to be obtained, depending on the aluminum compound used, always with molecular weight and gel values suitable for the industrial applications specified.

## Claims

1. A process for the preparation of 1,4-cis polybutadiene by means of the polymerization of 1,3-butadiene, carried out in one or more solvents and in the presence of a catalytic system which comprises one or more catalysts selected from cobalt complexes, preformed or formed in situ, having general formula (I)
Co^{(z)}L_{b}Wₘ
wherein (z), the cobalt oxidation number, is 2 or 3;
b = 1, 2, 3;
m = 0, 1, 2;
W is selected from Br, Cl, acetylacetonate, carboxylate, with the constraint that:
when z = 2; m = 2, b = 1; W = Cl, Br, carboxylate;
when z = 3; m = 0, 1, 2; b = 1, 2, 3; W = acetylacetonate;
**characterized in that**:
L is a ligand having general formula (Ia) 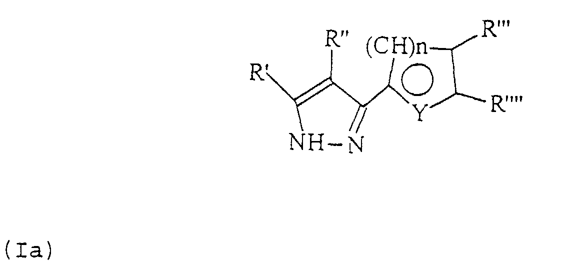 wherein
Y is selected from N, O and S
R' and R'', the same or different, are selected from hydrogen, linear or branched alkyl groups containing from 1 to 10 carbon atoms, cycloalkyl groups and aryl groups containing from 6 to 20 carbon atoms, wherein the above groups can be optionally halogenated, preferably with fluorine;
R''', R'''', the same or different, are selected from hydrogen, halogen, linear or branched C₁-C₂₀ alkyl groups, C₆-C₂₀ aryl groups, or they jointly form a condensed benzene ring, wherein said aryl groups or said condensed benzene ring can be optionally substituted with linear or branched alkyl groups containing from 1 to 10 carbon atoms,
n is an integer having the value of 1 or 2.

2. The process according to claim 1, wherein W is selected from Cl and acetylacetonate.

3. The process according to claim 1, wherein R' and R'', the same or different, are selected from H, CH₃, CF₃.

4. The process according to claim 1, wherein R'''= R''' = H.

5. The process according to claim 1, wherein R''' and R'''' are jointly equal to -(-CH=CH-)₂-.

6. A catalytic system useful for the polymerization of 1,3 butadiene comprising:
(a) a complex preformed or formed in situ having general formula (I) CO^{(z)}L_{b}Wₘ wherein z, L, b, m, W have the meanings defined above;
(b) organo-derivatives of aluminum selected from:
(b1) aluminoxanes and relative derivatives,
(b2) halogen aluminum alkyls selected from (III) AlRₙX₃₋ₙ (with n = 1 or 2) and (IV) Al₂RₙX₆₋ₙ (n = 1-5),
wherein R is a C₁-C₂₀ alkyl group, X is chlorine or bromine.

7. The catalytic system according to claim 6, wherein R is a C₁-C₅ alkyl group, X is chlorine or bromine.

8. A process for obtaining linear 1,4-cis polybutadiene by means of the polymerization of 1,3-butadiene in the presence of one or more solvents and a catalytic system, **characterized in that** the catalytic system comprises:
(a) a catalyst selected from one or more cobalt complexes, preformed or formed in situ, having general formula (I) Co^{(z)}L_{b}Wₘ, wherein z, b, m, L, W have the meanings defined above;
(b) a cocatalyst selected from aluminoxanes and relative derivatives.

9. A process for obtaining branched 1,4-cis polybutadiene by means of the polymerization of 1,3-butadiene in the presence of one or more solvents and a catalytic system, **characterized in that** the catalytic system comprises:
(a) a catalyst selected from one or more cobalt complexes, preformed or formed in situ, having general formula (I) Co^{(z)}L_{b}Wₘ, wherein z, b, m, L, W have the meanings defined above;
(b) a cocatalyst selected from halogen derivatives having general formula (III) AlRₙX₃₋ₙ (with n = 1 or 2) or (IV) Al₂RₙX₆₋ₙ (n = 1-5), wherein R is a C₁-C₂₀ alkyl group, X is chlorine or bromine, preferably chlorine.

10. The process for the preparation of 1,4-cis polybutadiene according to claims 8 and 9, **characterized in that** the molar ratio between the complex having general formula (I) and the aluminum of the cocatalyst ranges from 1:5 to 1:10000.

11. The process according to claim 10, wherein the molar ratio between the complex having general formula (I) and the aluminum of the cocatalyst ranges from 1:10 to 1:1000.

12. The process according to claims 8 and 9, **characterized in that** the molar ratio between the complex having general formula (I) and 1,3-butadiene ranges from 1/10⁵ to 1/10².

13. The process according to claim 12, **characterized in that** the molar ratio between the complex having general formula (I) and 1,3-butadiene ranges from 1/2x10⁴ to 1/10³.

14. The process according to claims 8 and 9, wherein, when the formation in situ technique of the complex having general formula (I), is used, it is possible to adopt a molar ratio between ligand (L) and cobalt salt ranging from at least 1/1 to 3/1.

15. The process according to claims 8 and 9, **characterized in that** the solvent is selected from aliphatic hydrocarbons, olefins included, cycloaliphatic, aromatic hydrocarbons and relative mixtures.

16. The process according to claim 15, **characterized in that** the solvent is selected from hydrocarbons selected from within the group of C₄ to C₈ aliphatic hydrocarbons, olefins included, within the group of C₅ to C₁₀ cycloaliphatic hydrocarbons, and relative mixtures.

17. The process according to claims 8 and 9, wherein the temperature ranges from -30°C to +60°C.

18. The process according to claim 17, wherein the temperature-ranges from -10°C to +40°C.
